# FASCICULE DE BREVET EUROPEEN

(11) **EP 1 537 419 B1**
(45) Date de publication et mention de la délivrance du brevet: **01.06.2011**
(21) Numéro de dépôt: 03773780.6
(22) Date de dépôt: 15.09.2003
(51) Int. Cl.: G01N 33/68, C12Q 1/02, G01N 33/80, G01N 33/53, A61K 39/395

(54) **Procédé d'évaluation de l'efficacité ADCC médiée par le CD16 d'anticorps monoclonaux ou polyclonaux**
Test für CD16-vermittelte ADCC Effektivität von monoklonalen oder polklonalen Antikörpern
Test for CD16-mediated ADCC efficacy of monoclonal or polyclonal antibodies

(30) Priorité: 13.09.2002 FR 0211415; 13.09.2002 FR 0211416
(43) Date de publication de la demande: 08.06.2005
(60) Demande divisionnaire: 09152731.7
(73) Titulaire: LFB Biotechnologies, 91940 Les Ulis (FR)
(72) Inventeur: DE ROMEUF, Christophe, F-59000 Lille (FR); GAUCHER, Christine, F-59320 Sequedin (FR); GLACET, Arnaud, F-59147 Gondecourt (FR); DHAINAUT, Frédéric, F-91870 Boissy-le-Sec (FR); BOUREL, Dominique, F-59110 La Madeleine (FR); BIHOREAU, Nicolas, F-91400 Orsay (FR); NONY, Emmanuel, F-92160 Antony (FR)
(74) Mandataire: Le Forestier, Eric
(86) Numéro de dépôt international: PCT/FR2003/002715
(87) Numéro de publication internationale: WO 2004/024768

(56) Documents cités:
- WO-A-00/59540
- WO-A-01/77181
- CROWE J S ET AL: "HUMANIZED MONOCLONAL ANTIBODY CAMPATH-1H MYELOMA CELL EXPRESSION OF GENOMIC CONSTRUCTS NUCLEOTIDE SEQUENCE OF CDNA CONSTRUCTS AND COMPARISON OF EFFECTOR MECHANISMS OF MYELOMA AND CHINESE HAMSTER OVARY CELL-DERIVED MATERIAL" CLINICAL AND EXPERIMENTAL IMMUNOLOGY, vol. 87, no. 1, 1992, pages 105-110, XP008034008 ISSN: 0009-9104
- VIVIER E ET AL: "SIGNALING FUNCTION OF RECONSTITUTED CD16:ZETA:GAMMA RECEPTOR COMPLEX ISOFORMS" INTERNATIONAL IMMUNOLOGY, OXFORD UNIVERSITY PRESS, GB, vol. 4, no. 11, 1992, pages 1313-1323, XP009011864 ISSN: 0953-8178
- ROUARD H ET AL: "FC RECEPTORS AS TARGETS FOR IMMUNOTHERAPY" INTERNATIONAL REVIEWS OF IMMUNOLOGY, HARWOOD ACADEMIC PUBLISHERS, LONDON, GB, vol. 16, no. 1/2, 1997, pages 147-185, XP001010510 ISSN: 0883-0185
- GRUEL N ET AL: "BYPASSING TUMOR-SPECIFIC AND BISPECIFIC ANTIBODIES: TRIGGERING OF ANTITUMOR IMMUNITY BY EXPRESSION OF ANTI-FCGAMMAR SCFV ON CANCER CELL SURFACE", GENE THERAPY, MACMILLAN PRESS LTD., BASINGSTOKE, GB, vol. 8, no. 22, 1 November 2001 (2001-11-01), pages 1721-1728, XP009011865, ISSN: 0969-7128, DOI: DOI:10.1038/SJ.GT.3301575
- DE ROMEUF CHRISTOPHE ET AL: "Chronic lymphocytic leukaemia cells are efficiently killed by an anti-CD20 monoclonal antibody selected for improved engagement of FcgammaRIIIA/CD16.", BRITISH JOURNAL OF HAEMATOLOGY MAR 2008 LNKD- PUBMED:18302712, vol. 140, no. 6, March 2008 (2008-03), pages 635-643, ISSN: 1365-2141

## Description

La présente demande concerne un procédé pour mesurer l'activation d'une cellule effectrice, appartenant au système immunitaire ou modifiée in vitro par un anticorps monoclonal (AcMo) ou polyclonal, caractérisé en ce qu'il comprend une mise en contact de cellules exprimant le récepteur CD16 dans un milieu réactionnel en présence de l'anticorps et de l'antigène dudit anticorps et une mesure de la quantité d'au moins une cytokine produite par la cellule exprimant le récepteur CD16. La demande porte également la sélection d'anticorps présentant la caractéristique d'induire l'expression de cytokines et d'interleukines, notamment l'IFNγ ou l'IL2.

L'immunothérapie à l'aide d'anticorps polyclonaux ou monoclonaux est en passe de devenir un des aspects les plus importants de la médecine. En revanche, les résultats obtenus lors d'essais cliniques apparaissent contrastés. En effet, il peut s'avérer que l'anticorps monoclonal ne soit pas suffisamment efficace. Aujourd'hui, la recherche s'oriente sur le fragment Fcγ de l'immunoglobuline afin d'améliorer les propriétés des anticorps. A terme, cela devrait permettre l'obtention d'anticorps qui interagissent et activent les récepteurs des cellules effectrices (macrophage, lymphocyte T et NK).

L'activité biologique de certaines immunoglobulines G est dépendante de la structure des oligosaccharides présents sur la molécule, et notamment sur sa partie Fc. Les molécules IgG de toutes les sous-classes humaines et murines possèdent un N-oligosaccharide fixé au domaine CH₂ de chaque chaîne lourde (au résidu Asn 297 pour les IgG humaines). L'influence de ce résidu glycannique sur la capacité de l'anticorps à interagir avec des molécules effectrices (Fc récepteurs et complément) a été démontrée. L'inhibition de glycosylation d'une IgG1 humaine, par culture en présence de Tunicamycine, provoque par exemple une diminution de 50 fois de l'affinité de cet anticorps pour le récepteur FcγRI présent sur les monocytes et macrophages (Leatherbarrow et al, 1985). La fixation au récepteur FcγRIII est également affectée par la perte de carbohydrates sur l'IgG, puisqu'il a été décrit qu'une IgG3 non glycosylée est incapable d'induire une lyse de type ADCC par l'intermédiaire du récepteur FcγRIII des cellules NK (Lund et al, 1990).

Mais, au-delà de la présence nécessaire de ces résidus glycanniques, c'est plus précisément l'hétérogénéité de leur structure qui peut aboutir à des différences dans la capacité à engager des fonctions effectrices. Des profils de galactosylation variables en fonction des individus (IgG1 humaines sériques) ont été observés. Ces différences reflètent probablement des disparités dans l'activité des galactosyltransférases et autres enzymes entre les clones cellulaires de ces individus (Jefferis et al, 1990). Alors que cette hétérogénéité normale des processus post-traductionnels génère différentes glycoformes (même dans le cas d'anticorps monoclonaux), elle peut conduire à des structures atypiques associées à certains états pathologiques comme l'arthrite rhumatoïde, la maladie de Crohn, pour lesquelles une proportion importante de résidus agalactosylés a été mise en évidence (Parekh et al, 1985).

Devant la complexité posée par la relation existante entre les différentes structures glycanniques et l'activité des anticorps, il serait utile de pouvoir discriminer rapidement quels sont les anticorps efficaces et permettre ainsi de sélectionner des lignées cellulaires produisant des anticorps ayant une meilleure efficacité ou des propriétés spécifiques dans l'activation ou l'inhibition de certains composants du système immunitaire.

Dans la demande FR 0004685 du 12 avril 2000 (LFB), nous avions décrit un nouveau procédé de préparation d'un anticorps monoclonal capable d'activer les cellules effectrices exprimant le FcγRIII. Dans ce procédé, on teste des anticorps monoclonaux provenant d'hybridomes ou de lignées transfectées dans un mélange réactionnel comprenant les cellules cibles desdits anticorps, des cellules effectrices comprenant des cellules exprimant le FcγRIII et des IgG polyvalentes. Ainsi, on peut déterminer le pourcentage de lyse des cellules cibles et sélectionner des anticorps monoclonaux qui activent les cellules effectrices provoquant une lyse significative des cellules cibles (activité ADCC de type FcγRIII). Par exemple, la partie Fab de l'anticorps anti-D va se fixer sur l'antigène Rhésus D porté par les hématies. Suite à cette fixation, sa partie Fc se fixe alors sur le récepteur Fc gamma RIII ou CD16 de la cellule effectrice (cellule NK). Ce « sandwich » induit la sécrétion de substances chimiques de type perforines qui vont lyser le globule rouge. Il s'agit donc d'une cytotoxicité cellulaire anticorps dépendante (CCDA) ou ADCC en anglais. Pour se rapprocher des conditions physiologiques, le test est effectué en présence d'immunoglobulines polyvalentes humaines.

Dans le cadre de l'invention, on a trouvé que la fixation d'un anticorps sur son ligand peut induire une activation de la cellule Jurkat transfectée CD16 induisant la sécrétion d'IL2. Une forte corrélation est observée entre la sécrétion d'IL2 par Jurkat CD16 et l'activité ADCC médiée par le CD16 des cellules effectrices. En outre, nous avons observé qu'un même anticorps dirigé contre un antigène donné soit complètement inefficace lors qu'il est produit dans des lignées de myélome de souris, alors qu'il se montre très efficace lors qu'il est produit dans d'autres lignées cellulaires.

Le problème est donc de savoir quelle est la capacité d'un anticorps donné à stimuler la production de cytokines par les cellules effectrices et quelles sont les conséquences d'une telle activation en fonction de la nature des cytokines libérées.

La demande propose donc l'utilisation d'anticorps sélectionnés par un test Jurkat CD16 par mesure d'IL2 sécrétée ou d'autres cytokines, ce qui permet de garantir l'activité biologique desdits anticorps pour un usage thérapeutique.

L'invention est telle que décrite dans les revendications.

La présente demande se rapporte à un procédé pour mesurer l'activation d'une cellule effectrice appartenant au système immunitaire, transformée ou non, par un anticorps monoclonal (AcMo) ou polyclonal, caractérisé en ce qu'il comprend une mise en contact de cellules exprimant le récepteur CD16 dans un milieu réactionnel en présence de l'anticorps et de l'antigène dudit anticorps et une mesure de la quantité d'au moins une cytokine produite par la cellule exprimant le récepteur CD 16.

On entend par cellule transformée, une cellule modifiée génétiquement de sorte à exprimer un récepteur, en particulier le récepteur CD16.
D'une façon générale, on utilise pour la sélection des anticorps une lignée de type Jurkat ou une autre lignée transfectée avec un vecteur d'expression codant pour récepteur Fc, incluant CD16, CD32 et CD64, comme cellule effectrice. De préférence, on utilise une lignée Jurkat transfectée avec un vecteur d'expression codant pour le récepteur CD16 comme cellule effectrice. Cette lignée est particulièrement avantageuse car elle est immortalisée et se développe indéfiniment dans des milieux cultures.

Parmi les cytokines que l'on peut quantifier, il est possible de mesurer la production d'au moins une cytokine sélectionnée parmi IL-1, IL-2, IL-3, IL-4, IL-5, IL-6, IL-7, IL-8, IL-9, IL-10,... TNFa, TGFβ, IP10 et IFNγ. On peut choisir avantageusement l'interleukine IL-2.

Le taux de cytokine produite est un marqueur d'activation ou d'inhibition des cellules effectrices.

De préférence le taux d'interleukine IL2 sécrétée reflète la qualité de l'anticorps fixé par le récepteur CD16 quant à son intégrité (fonction Fc) et à son efficacité (site antigénique) de liaison à l'antigène. La mesure du taux d'IL2 est corrélée à une activité du type ADCC.

Dans un autre aspect, la demande concerne un procédé pour évaluer l'efficacité d'un anticorps monoclonal ou polyclonal, caractérisé en ce qu'il comprend une mise en contact de cellules effectrices du système immunitaire, transformées ou non, exprimant le récepteur CD16 dans un milieu réactionnel en présence d'un anticorps et de l'antigène dudit anticorps et une mesure de la quantité d'au moins une cytokine produite par la cellule exprimant le récepteur CD 16.
Ce procédé est particulièrement adapté pour évaluer l'efficacité d'un anticorps monoclonal ou polyclonal de spécificité anti-Rh D du globule rouge humain.

Dans un autre aspect, la demande concerne un procédé pour évaluer la capacité d'une cellule à produire un anticorps monoclonal efficace, caractérisé en ce qu'il comprend une mise en contact de cellules effectrices du système immunitaire, transformées ou non, exprimant le récepteur CD16 dans un milieu réactionnel en présence d'un anticorps et de l'antigène dudit anticorps et une mesure de la quantité d'au moins une cytokine produite par la cellule exprimant le récepteur CD16.

Ce procédé peut être mis en oeuvre pour des cellules utilisées pour la production d'anticorps thérapeutiques, telles que CHO, YB2/0, des cellules lymphoblastoïdes humaines, des cellules d'insectes et des cellules de myélomes murines.

Ce procédé peut également être appliqué à l'évaluation de la production d'AcMo par des plantes transgéniques ou de mammifères transgéniques.

Dans un aspect complémentaire, la demande vise un procédé pour évaluer l'efficacité et l'intégrité d'anticorps polyclonaux après une étape ou plusieurs étapes de purification, caractérisé en ce qu'il comprend une mise en contact de cellules effectrices du système immunitaire, transformées ou non, exprimant le récepteur CD16 dans un milieu réactionnel en présence de l'anticorps purifié et de l'antigène dudit anticorps et une mesure de la quantité d'au moins une cytokine produite par la cellule exprimant le récepteur CD 16.

Les procédés décrits ci-dessus peuvent éventuellement être réalisés en présence d'immunoglobulines humaines (IVIg).

A titre d'exemple, on sélectionnera les anticorps pour lesquels une augmentation supérieure à 100%, 250%, 500%, ou 1000% du taux de libération d'IL-2 est observée par rapport au contrôle en absence d'anticorps ou un anticorps donné comme référence négative.

La demande vise également l'utilisation du procédé décrit ci-dessus pour sélectionner des anticorps efficaces pour un traitement thérapeutique. Par exemple, l'anticorps sélectionné peut être un anti-D. Il peut également être destiné au traitement des maladies autoimmunes et inflammatoires, des cancers et des infections par des agents pathogènes.

La demande porte également sur un kit permettant d'évaluer l'activité biologique d'un anticorps comprenant des moyens et des réactifs nécessaires et des cellules effectrices exprimant le récepteur CD16 pour la mise en oeuvre du procédé décrit ci-dessus permettant le dosage d'au moins une cytokine, notamment IL-2, IFN et TNF.

En outre, ce test peut également comprendre un test ADCC. A ce titre, la demande porte sur un procédé de sélection d'un anticorps monoclonal chimérique, humanisé ou humain optimisé caractérisé en ce qu'il comprend une mise en contact de cellules exprimant le récepteur CD16 dans un milieu réactionnel en présence de l'anticorps et de l'antigène dudit anticorps et une mesure de la quantité d'au moins une cytokine produite par la cellule exprimant le récepteur CD16.

Dans un mode de réalisation particulier, l'anticorps de la demande est capable d'induire la sécrétion d'au moins une cytokine par une cellule leucocytaire, en particulier de la famille des NK (natural killer) ou par des cellules du groupe monocytes-macrophages. D'une façon générale, on utilise pour la sélection des anticorps une lignée de type Jurkat ou une autre lignée transfectée avec un vecteur d'expression codant pour récepteur Fc, incluant CD16, CD32 et CD64, comme cellule effectrice. De préférence, on utilise pour la sélection des anticorps une lignée Jurkat transfectée avec un vecteur d'expression codant pour le récepteur CD16 comme cellule effectrice. Cette lignée est particulièrement avantageuse car elle est immortalisée et se développe indéfiniment dans des milieux cultures. Le taux d'interleukine IL2 sécrétée reflète la qualité de l'anticorps fixé par le récepteur CD16 quant à son intégrité (fonction Fc) et à son efficacité (site antigénique) de liaison à l'antigène.

Dans un autre mode de réalisation, l'anticorps optimisé peut être préparé après avoir été purifié et/ou modifié ex vivo par modification de la structure glycannique du fragment Fc. A cet effet, on peut utiliser tout moyen chimique, chromatographique ou enzymatique approprié pour modifier la structure glycannique des anticorps.

Dans un autre mode de réalisation, l'anticorps peut être produit par des cellules de lignées de myélome de rat, en particulier YB2/0 et ses dérivées. D'autres lignées peuvent être sélectionnées pour leurs propriétés de produire les anticorps définis ci-dessus. On pourra tester par exemple des cellules lymphoblastoïdes humaines, des cellules d'insectes et des cellules de myélome murin. La sélection peut également être appliquée à l'évaluation des anticorps produits par des plantes transgéniques ou de mammifères transgéniques. A cet effet, la production dans CHO sert de référence (CHO étant employée pour la production d'anticorps médicament) pour comparer et sélectionner les systèmes de production conduisant aux anticorps selon la demande.

La structure glycannique générale de l'anticorps est de type biantenné, avec des chaînes courtes, une faible sialylation, des mannoses et GlcNAc du point d'attache terminaux non intercalaires, et une faible fucosylation. Dans ces anticorps, le taux de GlcNac intermédiaire est non nul. Par exemple, on peut utiliser des compositions d'anticorps présentant une teneur supérieure à 60%, de préférence supérieure à 80% pour les formes G0 + G1 + G0F + G1F étant entendu que les formes G0F + G1F sont inférieures à 50%, de préférence inférieures à 30%. Ces compositions peuvent être obtenues avec des lignées de myélomes de rat, par exemple avec la lignée YB2%.

Dans un deuxième aspect, la demande vise l'utilisation d'un anticorps décrit ci-dessus pour la préparation d'un médicament destiné à induire la sécrétion d'au moins une cytokine par une cellule effectrice appartenant au système immunitaire, ledit anticorps étant caractérisé en ce qu'il est susceptible d'être obtenu par un procédé de sélection comprenant une mise en contact de cellules effectrices du système immunitaire exprimant le récepteur CD16, transformées ou non, dans un milieu réactionnel en présence de l'anticorps à tester et de l'antigène dudit anticorps et une mesure de la quantité d'au moins une cytokine produite par la cellule exprimant le récepteur CD16.

De préférence, on utilise pour la sélection des anticorps une lignée Jurkat transfectée avec un vecteur d'expression codant pour le récepteur CD16 comme cellule effectrice. Lesdites cytokines libérées sont des interleukines, des interférons et des facteurs de nécrose tissulaire (TNF). Ainsi, l'anticorps sélectionné a la capacité d'induire la sécrétion d'au moins une cytokine choisie parmi IL-1, IL-2, IL-3, IL-4, IL-5, IL-6, IL-7, IL-8, IL-9, IL-10,... TNFa, TGFβ, IP10 et IFNγ par les cellules effectrices du système immunitaire exprimant le récepteur CD16.

De préférence, l'anticorps sélectionné a la capacité d'induire la sécrétion d'IL-2 par les cellules effectrices du système immunitaire exprimant le récepteur CD16. Le taux d'interleukine IL2 sécrétée reflète la qualité de l'anticorps fixé par le récepteur CD16 quant à son intégrité (fonction Fc) et à son efficacité (site antigénique) de liaison à l'antigène. La mesure du taux d'IL2 est corrélée à une activité du type ADCC.

La sélection peut se faire sur des anticorps produits par des cellules couramment utilisées pour la production d'anticorps thérapeutiques, telles que CHO, YB2/0, les cellules lymphoblastoïdes humaines, les cellules d'insectes et les cellules de myélomes murines. La sélection peut également être appliquée à l'évaluation d'anticorps produits par des plantes transgéniques ou de mammifères transgéniques.

De préférence, la demande vise l'utilisation d'un anticorps produit par une lignée de myélome de rat, par exemple avec la lignée YB2/0 pour la préparation d'un médicament destiné à induire la sécrétion d'au moins une cytokine par une cellule effectrice appartenant au système immunitaire. A ce titre, la demande se rapporte à l'utilisation d'un anticorps présentant une structure glycannique de type biantenné, avec des chaînes courtes, une faible sialylation, des mannoses et GIcNAc du point d'attache terminaux non intercalaires, et une faible fucosylation pour la préparation d'un médicament destiné à induire la sécrétion d'au moins une cytokine par une cellule effectrice appartenant au système immunitaire. Dans cet anticorps, le taux de GlcNac intermédiaire est non nul. Par exemple, on peut utiliser des compositions d'anticorps présentant une teneur supérieure à 60%, de préférence supérieure à 80% pour les formes G0 + G1 + G0F + G1F étant entendu que les formes G0F + G1F sont inférieures à 50%, de préférence inférieures à 30%.

Dans un mode de réalisation particulier, l'anticorps sélectionné est capable d'induire la sécrétion d'au moins une cytokine par une cellule leucocytaire, en particulier de la famille des NK (natural killer) ou par des cellules du groupe monocytes-macrophages.

La demande se rapporte également à l'utilisation des anticorps sélectionnés décrits ci-dessus spécifiques d'un antigène qui provient d'une cellule pathologique ou d'un organisme pathogène pour l'homme.
Cet anticorps est un anticorps monoclonal ou polyclonal.

Par exemple, l'anticorps est un anticorps monoclonal ou polyclonal de spécificité anti-Rhésus du globule rouge humain.

L'anticorps selon la demande peut également être un anticorps dirigé contre des virus pathogènes pour l'homme, contre des antigènes de tumeurs malignes ou contre les antigènes d'une bactérie ou d'un parasite pathogène pour l'homme.

Avantageusement, l'anticorps sélectionné montre une augmentation supérieure à 100%, 250%, 500%, ou 1000% du taux de libération d'IL-2 par rapport au contrôle en absence d'anticorps ou en présence d'un anticorps donné comme référence négative. Les procédés décrits ci-dessus peuvent éventuellement être réalisés en présence d'immunoglobulines humaines (IVIg). Pour la comparaison, des anticorps homologues produits dans CHO, ou encore des anticorps de référence disponibles dans le commerce peuvent être utilisés.

Dans un aspect supplémentaire, la demande vise l'utilisation desdits anticorps sélectionnés comme support thérapeutique en médecine humaine, notamment pour la fabrication d'un médicament destiné au traitement des maladies autoimmunes, inflammatoires, des cancers et des infections par des agents pathogènes.

### Légende

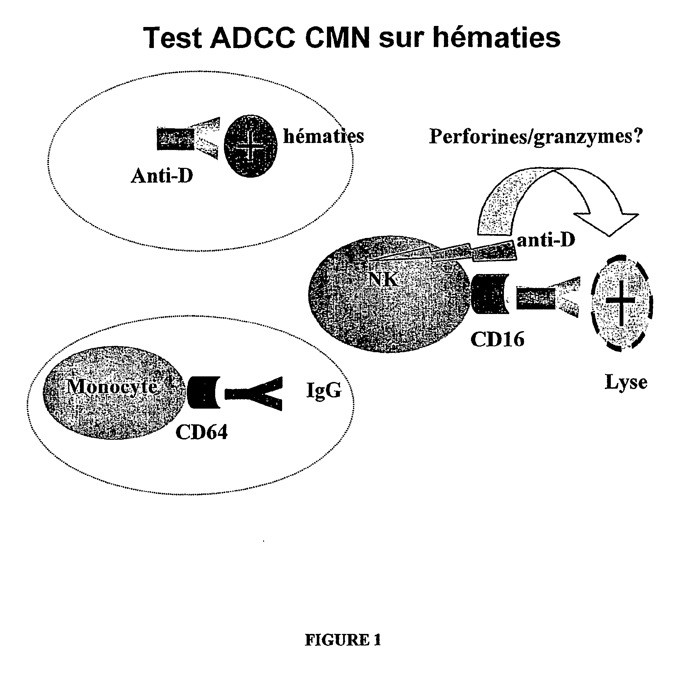

**Figure 1** **: Description du test ADCC CMN.**
   Les cellules mononucléées en présence de Tégéline (IVIg) sont incubées avec les anticorps anti-Rhésus D et des hématies Rhésus + (cible). Après une nuit à 37°C, on mesure la lyse des hématies par évaluation du taux d'hémoglobine relarguée dans le milieu réactionnel.

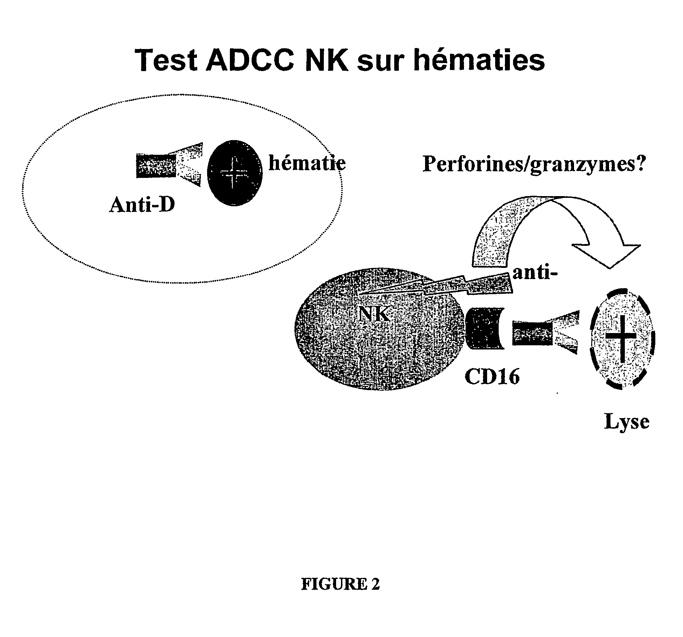

**Figure 2** **: Description du test ADCC NK**
   Les cellules NK purifiées sont incubées avec les anticorps anti-Rhésus D et des hématies Rhésus + (cible). Après une nuit à 37°C, on mesure la lyse des hématies par évaluation du taux d'hémoglobine relarguée dans le milieu réactionnel.

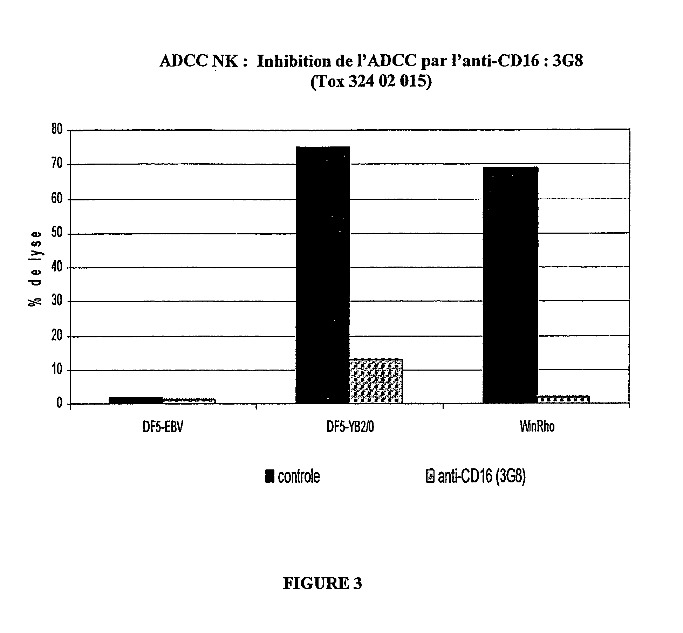

**Figure 3** **: Résultats ADCC NK et inhibition par l'anti-CD16 « 3G8».**
   Les anticorps anti-D, DF5-EBV (exprimé par la cellule B immortalisé EBV), DF5-YB2/0 (exprimé par la cellule YB2/0), sont comparés à l'anticorps polyclonal WinRho pour leur capacité à induire la lyse d'hématies Rhésus D en présence de cellules NK. L'inhibition de l'ADCC est étudiée en présence de l'anti-CD16 3G8.

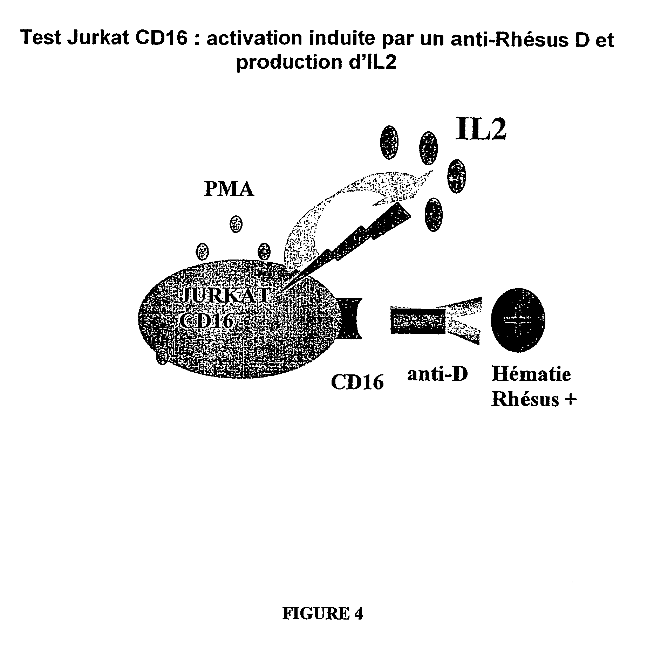

**Figure 4** **: Description du test Jurkat CD16.**
   Des cellules Jurkat CD16 sont mélangées avec différents anticorps anti-D en présence d'hématies Rhésus + et de PMA. Après une nuit d'incubation, la libération d'IL-2 dans le surnageant est quantifiée par ELISA.

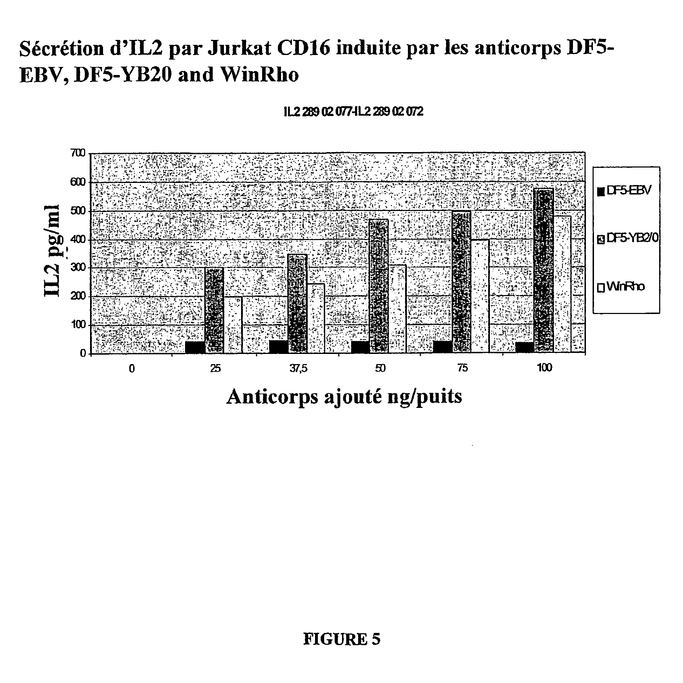

**Figure 5** **: Résultats du test Jurkat CD16.**
   Commentaires : les anticorps positifs en ADCC-NK induisent une sécrétion d'IL2 en présence de Jurkat CD16 et de leur cible.

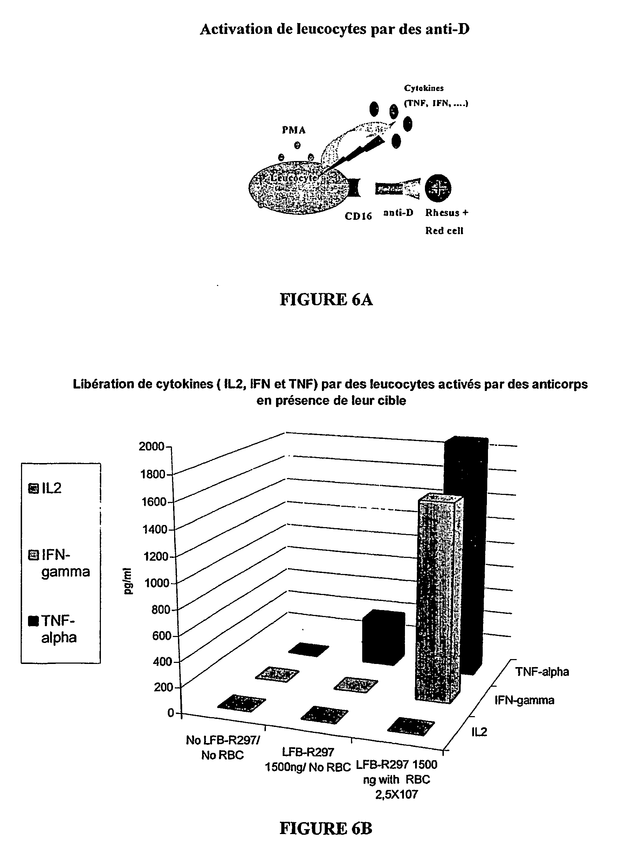

**Figure 6** **: Libération de cytokine (IL-2, IFN et TNF) par des leucocytes activés par des anticorps en présence de leur cible.**
   A- Schéma d'activation des leucocytes.
   B- Les leucocytes ont été incubés avec différents anticorps en présence d'hématies. Après une nuit d'incubation, la libération de TNFa et IFNγ dans le surnageant a été quantifiée par ELISA.

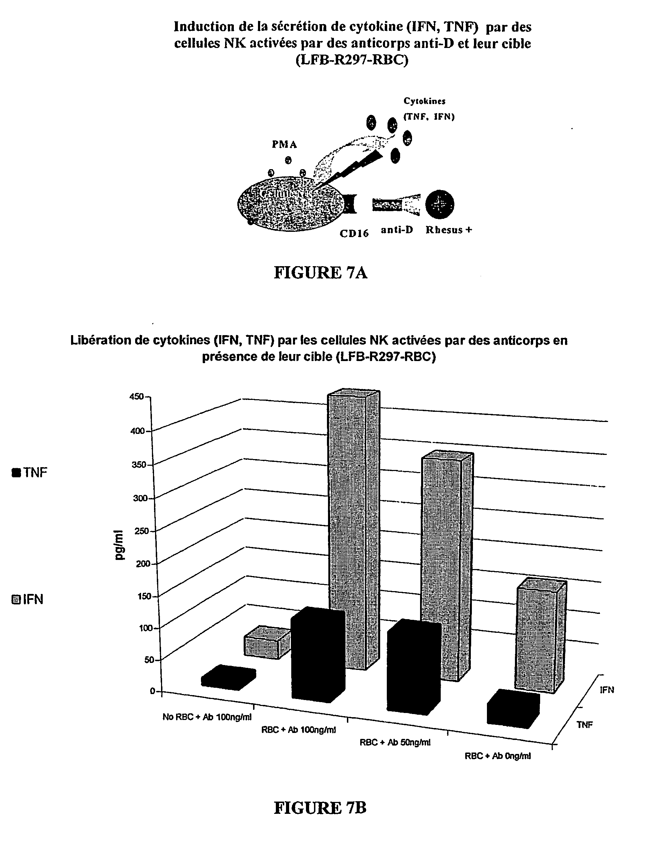

**Figure 7** **: Libération de cytokine (IFN, TNF) par des cellules NK activées par des anticorps en présence de leur cible (LFB-R297-RBC).** A- Schéma d'activation des cellules NK.
   B-Des cellules NK purifiées ont été mélangées avec différents anticorps anti-D en présence d'hématies Rhésus +. Après une nuit d'incubation, la libération de TNFa et d'IFNγ dans le surnageant a été quantifiée par ELISA.

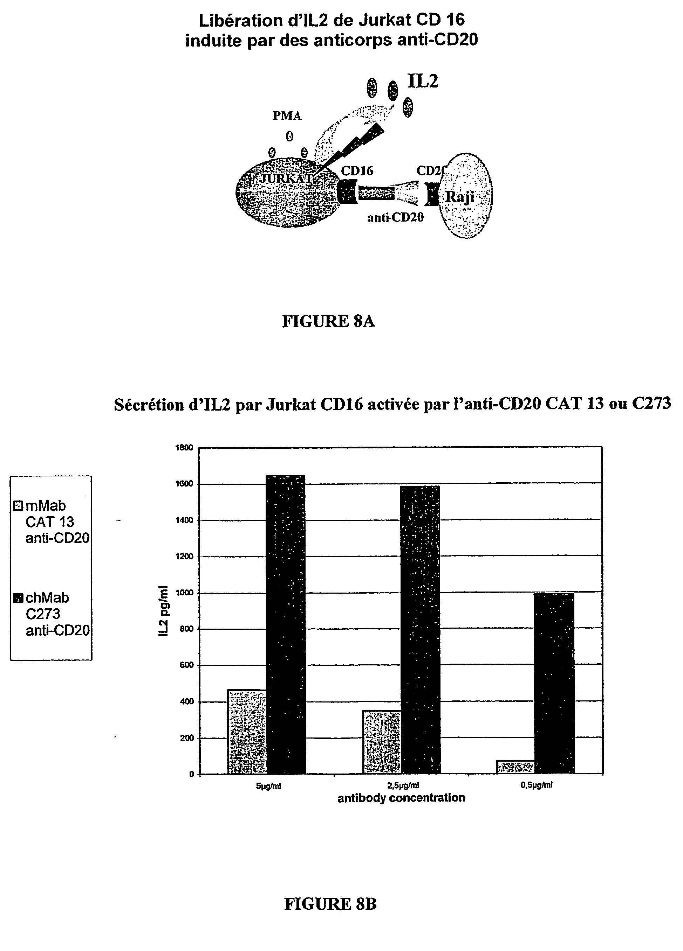

**Figure 8** **: Libération d'IL2 par Jurkat CD16 activées par un anti-CD20**
   A- Schéma d'activation de cellule Jurkat.
   B- Des cellules Jurkat CD16 ont été mélangées avec différents anticorps anti-CD20 (anticorps murin CAT13 et anticorps chimérique C273) en présence de cellules Raji et de PMA. Après une nuit d'incubation, la libération d'IL-2 dans le surnageant a été quantifiée par ELISA.

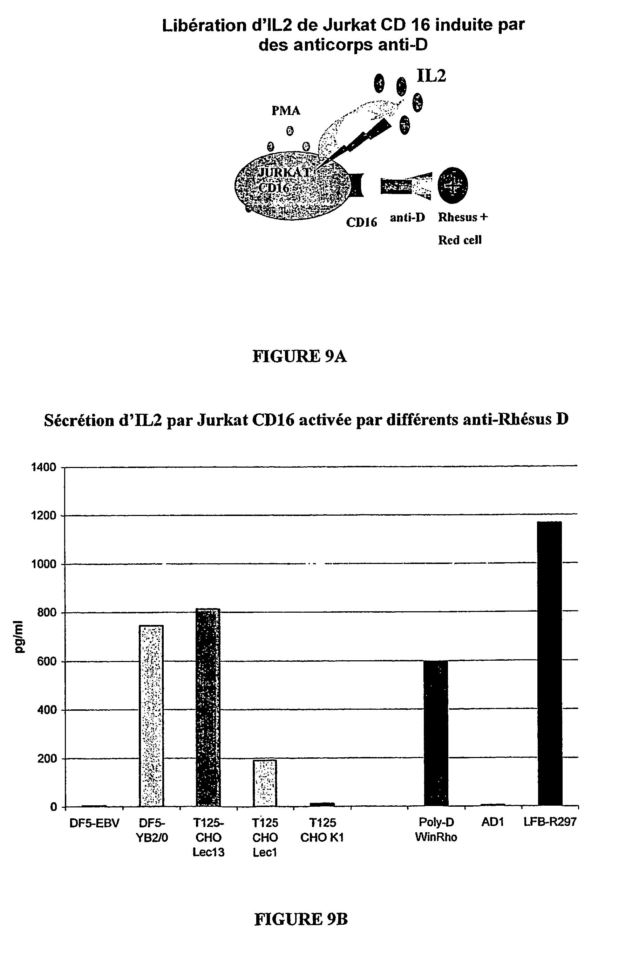

**Figure 9** **: Libération d'IL2 par Jurkat CD16 activées par un anti-D**
   A- Schéma d'activation de cellule Jurkat.
   B- Des cellules Jurkat CD16 ont été mélangées avec différents anticorps anti-D en présence d'hématies Rhésus + et de PMA. Après une nuit d'incubation, la libération d'IL-2 dans le surnageant a été quantifiée par ELISA. DF5 exprimé dans YB2/0 et T125 exprimé dans CHO Lec13 induisent une forte sécrétion d'IL2.

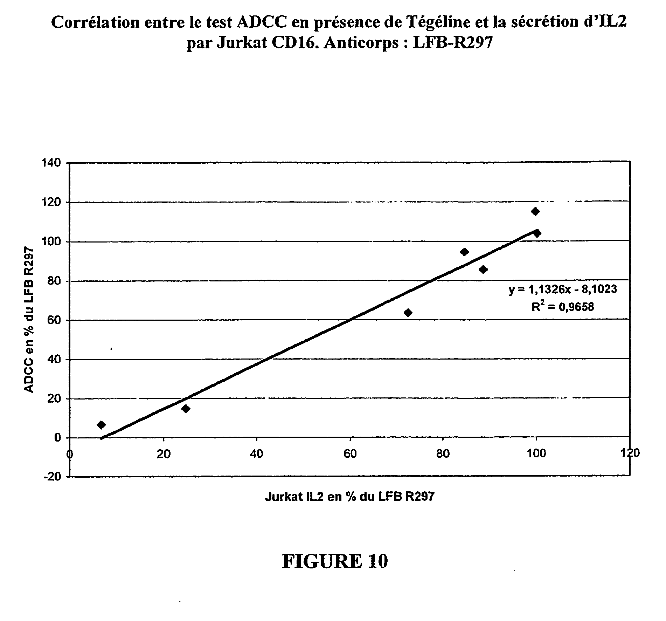

**Figure 10** **: Droite de corrélation entre l'ADCC (Tégéline 500µg/puits et anti-D à 7,5ng/puits) et le test Jurkat IL2.**

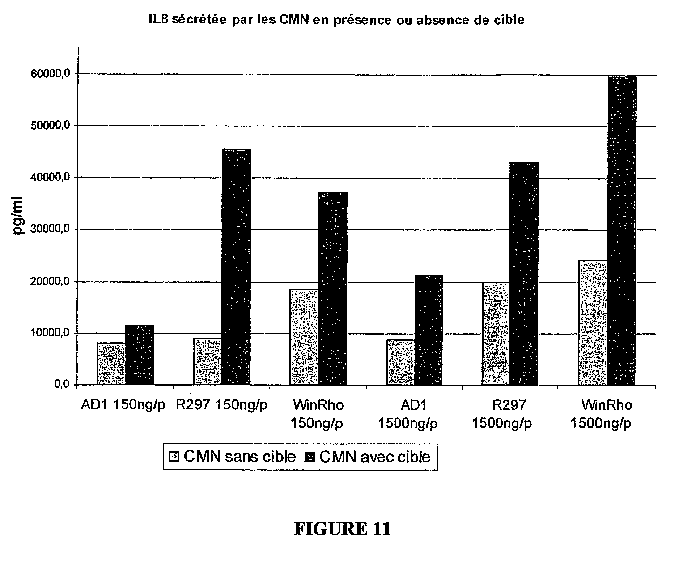

**Figure 11** **: Sécrétion d'IL-8 par les cellules mononuclées**

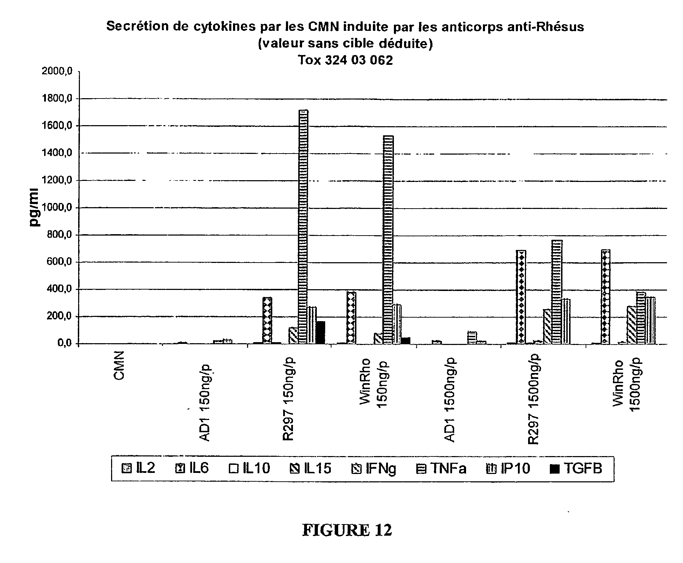

**Figure 12** **: Induction de la sécrétion de TNF alpha, IL-6 et TGF bêta par les cellules mononuclées**

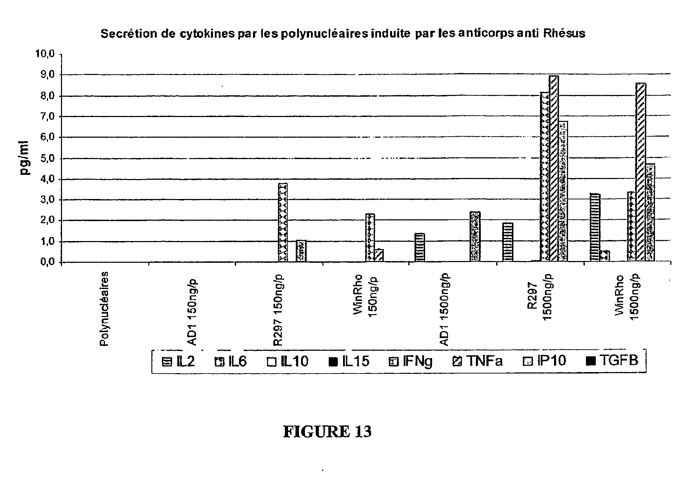

**Figure 13** **: Induction de la sécrétion de cytokines par les polynucléaires**

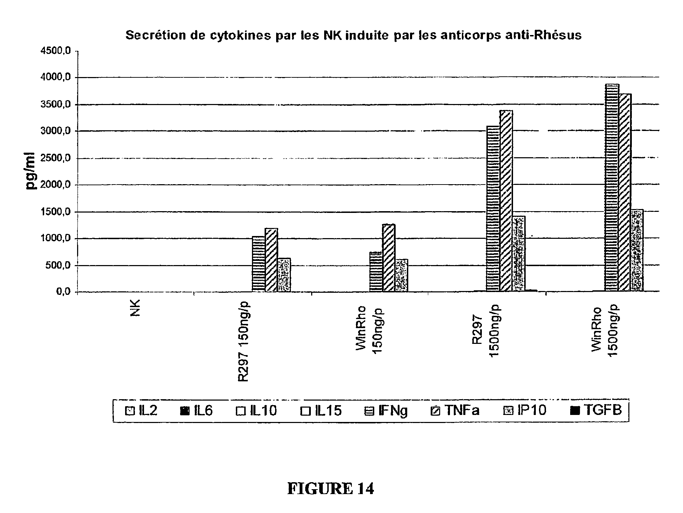

**Figure 14** **: Induction de la sécrétion de l'IFN gamma, TNF alpha et IP10 par les NK.**

### Exemple 1 : Test Jurkat CD16

### Anticorps :

Anticorps polyclonaux WinRho, anticorps monoclonal DF5-EBV, anticorps monoclonal DF5-YB2/0

### Principe :

Ce test évalue la capacité des anticorps anti-D à se fixer sur le récepteur CD16 (Fc gamma RIII) exprimé sur les cellules Jurkat CD16 et à induire la sécrétion d'IL2.
Ce test consiste à mettre en contact en plaque de 96 puits : les anticorps anti-D, les hématies Rhésus positif traitées à la papaïne, les cellules Jurkat CD16 et du PMA. Après une nuit d'incubation à 37°C, on centrifuge les P96 et on dose dans le surnageant la quantité d'IL2 secrétée.

### Matériel

Anticorps témoins positif : Poly-D WinRho, DF5 YB2/0.
Anticorps témoins négatifs : DF5
Hématies Rhésus positif
Cellules Jurkat CD 16
Kit dosage IL2 : Quantikine de chez R/D.

### Méthode

### Traitement à la papaïne des hématies.

1ml de culot d'hématies diluées en PBS incubé avec 1ml d'une solution de papaïne (1mg/ml) pendant 10mn à 37°C. Puis 3 lavages en H2O-NaCl 0.15M.
Mélange réactionnel :
- Anticorps : 50µl d'une dilution à 150ng/ml en IMDM 5% SVF
- PMA 50µl d'une dilution à 40ng/ml en IMDM 5% SVF
- Hématies traitées à la papaïne. 50µl à 8 10⁶/ml en IMDM 5% SVF
- Jurkat CD16. 50µl à 2x10⁶/ml en IMDM 5% SVF

Incubation 1 nuit à 37°C

Puis centrifugation des plaques, prélèvement de 100µl de surnageants et dosage d'IL2 avec le kit commercial. Lecture à 450nm.

On donne les valeurs (en pg/ml) sous forme d'histogramme pour chaque échantillon.

### Exemple 2 : Corrélation in vitro entre ADCC et libération d'IL-2 par Jurkat CD16.

Pour cette étude, 3 anticorps monoclonaux anti-D ont été comparés.
Mab DF5-EBV a été produit par des Lymphocytes B humains obtenus chez un donneur immunisé D-négatif et immortalisés par transformation avec EBV. Cet anticorps a été utilisé comme contrôle négatif étant donné qu'il a été montré qu'il est incapable d'éliminer les globules rouges Rhésus positif de la circulation lors d'un essai clinique.
L'anticorps monoclonal (Mab) DF5-YB2/0 a été obtenu en exprimant la séquence primaire de DF5-EBV dans la lignée YB2/0. L'anticorps monoclonal R297 et d'autres anticorps recombinants ont également été exprimés dans YB2/0.

Ces anticorps sont testés in vitro pour leur capacité à induire une lyse des globules rouges traités à la papaïne en utilisant des cellules mononucléées (PBL) comme effecteur.
Tous les tests ont été effectués en présence d'immunoglobulines humaines (IVIg) de sorte à reconstituer les conditions physiologiques.
On pense que les IVIg se lient avec une haute affinité au FcgammaRI (CD64). Les deux Mab DF5-YB2/0 et R297 induisent une lyse des globules rouges à un niveau comparable à celui des anticorps WinRho. En revanche, le Mab DF5-EBV est complètement inefficace.

Dans une deuxième série d'expérience, des cellules NK purifiées et des globules rouges non traités ont été utilisés comme effecteur et cibles respectivement. Après 5 heures d'incubation, les Mabs antiD-R297 et DF5-YB2/0 se sont montrés capables de provoquer la lyse des globules rouges, alors que DF5-EBV reste inefficace.
Dans ces deux expériences, la lyse des globules rouges a été inhibée par l'anticorps 3G8 dirigé contre le FcgammaRIII (CD16).

En résumé, ces résultats démontrent que l'ADCC provoquée par l'anticorps R297 et l'anticorps DF5-YB2/0 implique le FcgammaRIII exprimé à la surface des cellules NK.

Dans le cadre de l'invention, une troisième série d'expériences a mis en valeur un test in vitro à l'aide de cellule Jurkat CD16 pour évaluer l'efficacité d'anticorps anti-D. Les anticorps ont été incubés pendant la nuit avec des globules rouges Rhésus positif et des cellules Jurkat CD16. La libération d'IL-2 dans le surnageant à été évaluée par ELISA. Une forte corrélation entre l'ADCC et l'activation des cellules Jurkat a été observée, ce qui implique que ce test peut être utilisé pour faire la discrimination des anticorps anti-D en fonction de leur réactivité envers FcgammaRIII (CD 16).

Les mêmes échantillons sont évalués en ADCC et dans le test Jurkat IL2. Les résultats sont exprimés en pourcentage de l'anticorps de référence "LFB-R297". La courbe de corrélation entre les 2 techniques a un coefficient r2=0.9658 (figure 10).

En conclusion, ces données montrent l'importance des modifications post-traductionnelles de la structure des anticorps pour leur activité ADCC spécifique du FcgammaRIII. La libération de cytokines telles que l'IL-2 reflète cette activité.

### Exemple 3 : Activation de cellules NK et production d'IL2 et d'IFNγ

Modèle d'étude : Cellules NK purifiées à partir de sang périphérique. Applications : renforcement d'une réponse anti-tumorale. L'IL2 induit une activation des lymphocytes T et des cellules NK elles mêmes pouvant aller jusqu'à une stimulation de la prolifération cellulaire. L'IFNγ stimule l'activité des CTLs et peut renforcer l'activité des macrophages.

### Exemple 4 : Activation de monocytes macrophages et production de TNF et d'IL-IRa

Applications : renforcement de la phagocytose et induction de propriétés anti-inflammatoires. Le TNF stimule la prolifération des macrophages et des lymphocytes infiltrant les tumeurs. L'IL-1Ra est une cytokine produite par les macrophages qui entre en compétition avec l'IL1 au niveau de son récepteur et exerce ainsi un effet anti-inflammatoire.

### Exemple 5 : Activation de cellules dendritiques et production d'IL10

Applications : induction d'une tolérance spécifique à certains antigènes. L'IL10 est une molécule inhibitrice de l'activation de différentes cellules effectrices et de la production de cytokines.

### Exemple 6 : Induction de la sécrétion de cytokines par différentes cellules effectrices.

Trois populations cellulaires ont été étudiées : **les polynucléaires, les cellules mononucléées et les cellules NK.** Les synthèses de cytokines sont dépendantes de la présence de la cible. Il y a peu de différences dans les profils des cytokines induite par l'anticorps R297 et de l'anticorps polyclonal anti-D. AD1 est très souvent non inducteur de sécrétion de cytokines.

### Résultats :

6.1 L'anticorps monoclonal R297 et le polyclonal WinRho induisent une sécrétion importante d'IL8 en présence de cellules mononuclées. Cette sécrétion est dépendante de la concentration d'anticorps et de la présence de la cible antigénique. L'anticorps AD1 est beaucoup moins effecteur (figure 11), c'est à dire capable d'induire la production de cytokines.

Avec des cellules mononucléées (CMN), l'anticorps monoclonal R297 et le polyclonal WinRho induisent une sécrétion importante de TNF alpha, dans une moindre mesure bien que supérieure à AD1 une sécrétion d'IL6, d'IFN gamma, d'IP10, de TNF alpha et de TGF Béta . A la plus forte concentration d'anticorps, cette sécrétion d' IL6, IFN gamma, IP10 augmente , mais décroît pour le TNF alpha et le TGF Béta (figure 12).

6.2 L'anticorps monoclonal R297 et le polyclonal WinRho induisent une sécrétion très faible, mais supérieure à AD1, d'IL2, d'IFN gamma, de IP10 et de TNF par les polynucléaires. Cette sécrétion est dépendante de la concentration d'anticorps (figure 13).

6.3 L'anticorps monoclonal R297 et le polyclonal WinRho induisent une sécrétion importante d'IFN gamma, d'IP10 et de TNF par les cellules NK. Cette sécrétion est dépendante de la concentration d'anticorps (figure 14).

### Exemple 7 : Anticorps anti-CD 20 et anti-HLA DR chimériques optimisés produits dans YB2/0

### Introduction

Nos premiers résultats ont montré que les anticorps anti-D produits dans YB2/0 ainsi que les anticorps polyclonaux utilisés en clinique induisaient la production de cytokines, en particulier de TNF alpha et d'interféron gamma (IFN gamma) à partir de cellules NK purifiées ou de cellules mononucléées. Par contre d'autres anticorps anti-D, produits dans d'autres lignées cellulaires sont négatifs en ADCC et se sont révélés incapables d'induire cette sécrétion.

Les résultats complémentaires ci dessous montrent que ce mécanisme n'est pas exclusif aux anti-D en présence d'hématies Rhésus positif mais s'applique également aux anticorps anti-CD20 et anti-HLA DR exprimés dans YB2/0. L'expression dans CHO confère à l'anticorps des propriétés activatrices moins importantes. Ceci est en corrélation avec les résultats obtenus en ADCC.

### Matériel

### Anticorps.

Anti-CD20 : l'anticorps chimérique anti-CD20 transfecté dans YB2/0 est comparé à un anticorps commercial anti-CD20 produit dans CHO (Rituxan).

Anti-HLA DR : la même séquence codant pour l'anticorps chimérique anti-HLA DR est transfectée dans CHO (B11) ou YB2/0 (4B7).

*Cellules cibles* : cellules Raji exprimant à leur surface lesantigènes CD20 et HLA-DR *Cellule effectrices* : cellules NK humaines purifiées par sélection négative à partir de poche de sang humain.

### Méthode

Différentes concentrations d'anticorps anti-CD20 ou anti-HLA DR sont incubées avec les cellules Raji (cibles) et les cellules NK (effecteurs). Après 16 heures d'incubation, les cellules sont centrifugées. Les surnageants sont dosés en TNF alpha et IFN gamma.

### Résultats :

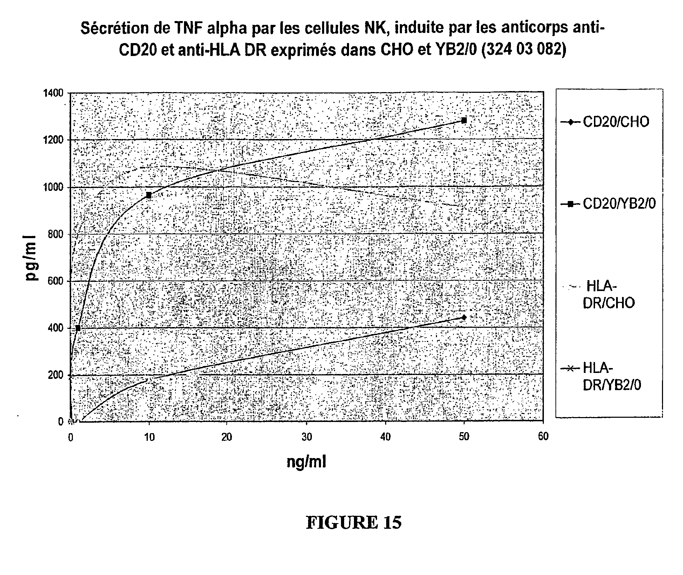

*7.1 TNF alpha* : Les résultats sont exprimés en pg/ml de TNF alpha dosé dans les surnageants. En abscisse figurent les différentes concentrations d'anticorps ajoutées dans le mélange réactionnel (figure 15).

Les anticorps anti-CD 20 et anti-HLA DR chimériques produits dans YB2/0 induisent des taux plus importants de TNF en présence de leur cible (Raji) par rapport aux mêmes anticorps produits dans CHO. La quantité de TNF alpha est bien dose dépendante de la concentration d'anticorps ajouté. A 10ng/ml d'anticorps on induit 5 fois plus de TNF alpha avec les anticorps produits dans YB2/0 par rapport aux anticorps produits dans CHO.

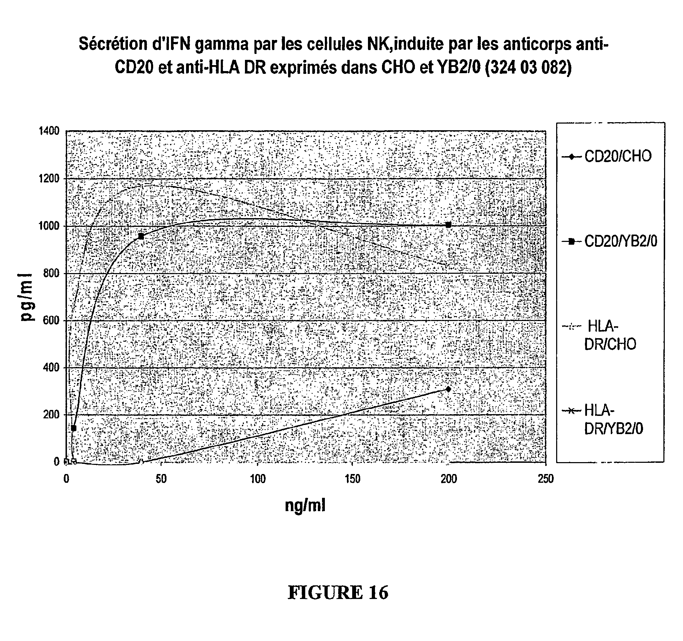

7.2 *IFN gamma*: Les résultats sont exprimés en pg/ml d'IFN gamma dosé dans les surnageants. En abscisse figurent les différentes concentrations d'anticorps ajoutées dans le mélange réactionnel (figure 16).

Les anticorps anti-CD 20 et anti-HLA DR chimériques produits dans YB2/0 induisent des taux plus importants d'IFN gamma en présence de leur cible (Raji) par rapport aux même anticorps produits dans CHO. La quantité d'IFN gamma est bien dose dépendante de la concentration d'anticorps ajouté. A toutes les concentrations utilisées (0 à 200ng/ml) l'anticorps anti-HLA DR produit dans CHO n'induit pas de sécrétion d'IFN gamma, alors que 40ng/ml de l'anticorps produit dans YB2/0 induit environ 1000pg/ml d'IFN gamma.

Pour l'anticorps anti-CD20, il faut moins de 10ng/ml de l'anticorps produit dans YB2/0 et 200ng/ml de l'anticorps produit dans CHO pour induire 300pg/ml d'IFN gamma (figure 16).

### REFERENCES

Jefferis, R., Lund, J., Mizutani, H., Nakagawa, H., Kawazoe, Y., Arata, Y. and Takahashi, N. A comparative study of the N-linked oligosaccharides structure of human IgG Subclass proteins. Biochem. J., 268 : 529-537 (1990).
Leatherbarrow, R.J., Rademacher, T.W., Dwek, R.A., Woof, J.M., Clark, A., Burton, D.R., Richardson, N. and Feinstein, A. Effector functions of monoclonal aglycosylated mouse IgG2a ; binding and activation of complement component C1 and interaction with human Fc receptor. Molec. Immun. 22, 407-415 (1985).
Lund, J., Tanaka, T., Takahashi, N., Sarmay, G., Arata, Y. and Jefferis, R. A protein structural change in aglycosylated IgG3 correlates with loss of hu Fcγ RI and hu FcγRIII binding and/or activation. Molec. Immun. 27,1145-1153 (1990).
Parekh, R.B., Dwek, R.A., Sutton, B.J., Fernandes, D.L., Leung, A., Stanworth, D., Rademacher, T.W., Mizuochi, T., Taniguchi, T., Matsuta, K., Takeuchi, F., Nagano, Y., Miyamoto, T. and Kobata, A. Asssociation of rheumatoid arthritis and primary osteoarthritis with changes in the glycosylation pattern of total serum IgG. Nature, 316 : 452-457 (1985).

## Revendications

1. Procédé in vitro pour évaluer l'efficacité ADCC médiée par le CD16 d'un anticorps monoclonal ou polyclonal, comprenant une mise en contact de cellules effectrices Jurkat transfectées avec un vecteur d'expression codant pour le récepteur CD16 dans un milieu réactionnel en présence dudit anticorps et de l'antigène dudit anticorps, et une mesure de la quantité d'IL-2 produite par les cellules effectrices Jurkat transfectées avec un vecteur d'expression codant pour le récepteur CD16, **caractérisé en ce que** la sécrétion d'IL-2 par les cellules Jurkat transfectées avec un vecteur d'expression codant pour le récepteur CD16 est corrélée avec l'activité ADCC médiée par le CD16.

2. Procédé selon la revendication 1, **caractérisé en ce que** l'anticorps est un anticorps polyclonal ou monoclonal de spécificité anti-Rh D du globule rouge humain.

3. Procédé selon l'un des revendications 1 à 2, **caractérisé en ce qu'**il comprend en outre une étape de sélection des anticorps pour lesquels une augmentation supérieure à 100%, 250%, 500%, ou 1000% du taux de libération d'IL-2 est observée par rapport au contrôle en absence d'anticorps ou en présence d'un anticorps donné comme référence négative.

4. Procédé selon l'une des revendications 1 à 3, **caractérisé en ce que** le mélange réactionnel comprend des immunoglobulines humaines (IVIg).

## Claims

1. In vitro method for evaluating the CD16-mediated ADCC efficacy of a monoclonal or polyclonal antibody, which comprises bringing together Jurkat effector cells transfected with an expression vector encoding the CD16 receptor in a reaction medium in the presence of said antibody and of the antigen for said antibody, and measuring the amount of IL-2 produced by the Jurkat effector cells transfected with an expression vector encoding the CD16 receptor, **characterized in that** the secretion of IL-2 by the Jurkat cells transfected with an expression vector encoding the CD16 receptor is correlated with the CD16-mediated ADCC activity.

2. Method according to Claim 1, **characterized in that** the antibody is a polyclonal or monoclonal antibody with anti-human red blood cell Rh D specificity.

3. Method according to either of Claims 1 and 2, **characterized in that** it also comprises a step of selecting the antibodies for which an increase of greater than 100%, 250%, 500% or 1000% in the level of IL-2 release is observed compared with the control in the absence of antibody or in the presence of an antibody given as negative reference.

4. Method according to one of Claims 1 to 3, **characterized in that** the reaction mixture comprises human immunoglobulins (IVIgs).

## Patentansprüche

1. In-vitro-Verfahren zur Bewertung der durch CD16 vermittelten ADCC-Wirksamkeit eines monoklonalen oder polyklonalen Antikörpers, umfassend das in-Kontakt-Bringen von Jurkat-Effektorzellen, die mit einem Expressionsvektor transfiziert sind, der für den CD16-Rezeptor codiert, in einem Reaktionsmedium in Anwesenheit des Antikörpers und des Antigens des Antikörpers, und das Messen der IL-2-Menge, die durch die Jurkat-Effektorzellen produziert wird, welche mit einem Expressionsvektor transfiziert sind, der für den CD16-Rezeptor codiert, **dadurch gekennzeichnet, dass** die Sekretion von IL-2 durch die Jurkat-Zellen, die mit einem Expressionsvektor transfiziert sind, der für den CD16-Rezeptor codiert, mit der durch CD16 vermittelten ADCC-Aktivität korreliert wird.

2. Verfahren nach Anspruch 1, **dadurch gekennzeichnet, dass** der Antikörper ein polyklonaler oder monoklonaler Antikörper ist, der die Spezifität anti-Rh D des menschlichen roten Blutkörperchens aufweist.

3. Verfahren nach einem der Ansprüche 1 bis 2, **dadurch gekennzeichnet, dass** es darüber hinaus einen Schritt der Selektion von Antikörpern umfasst, bei denen eine Zunahme der Freisetzungsmenge von IL-2 von mehr als 100 %, 250 %, 500 % oder 1000 % beobachtet wird, bezogen auf eine Kontrolle in Abwesenheit von Antikörper oder in Anwesenheit eines Antikörpers, der als negative Referenz gegeben ist.

4. Verfahren nach einem der Ansprüche 1 bis 3, **dadurch gekennzeichnet, dass** die Reaktionsmischung menschliche Immunglobuline (IVIg) umfasst.
